# EUROPEAN PATENT SPECIFICATION

(11) **EP 4 045 113 B1**
(45) Date of publication and mention of the grant of the patent: **10.12.2025**
(21) Application number: 20789143.3
(22) Date of filing: 13.10.2020
(51) Int. Cl.: A61M 5/24

(54) **CARTRIDGE FIXATION FOR A DRUG DELIVERY DEVICE**
KARTUSCHENFIXIERUNG FÜR EINE WIRKSTOFFABGABEVORRICHTUNG
FIXATION DE CARTOUCHE POUR DISPOSITIF D'ADMINISTRATION DE MÉDICAMENTS

(30) Priority: 15.10.2019 EP 19203413
(43) Date of publication of application: 24.08.2022
(73) Proprietor: Ypsomed AG, 3401 Burgdorf (CH)
(72) Inventor: HIRSCHEL, Jürg, 3007 Bern (CH); ALLENSPACH, Marcel, 3400 Burgdorf (CH); MOSIMANN, Manuel, 3600 Thun (CH); KALBERMATTER, Gabriel, 3400 Burgdorf (CH)
(74) Representative: Kleiner, Stefan
(86) International application number: PCT/EP2020/078700
(87) International publication number: WO 2021/074105

(56) References cited:
- EP-A1- 2 311 513
- US-A1- 2013 006 193
- US-A1- 2016 193 414
- US-A1- 2017 348 489
- US-B2- 9 132 237

## Description

### FIELD OF THE INVENTION

The present invention relates to medicament delivery devices for delivering, administering, dispensing, injecting, or infusing substances and/or liquids such as insulin or hormone preparations. The inventions departs from a housing and a cartridge holder connectable to the housing for holding a cartridge within the housing and the cartridge holder.

### BACKGROUND OF THE INVENTION

A variety of diseases exist that require regular treatment by parenteral subcutaneous or intramuscular administration of a drug or medicament, and a number of drug delivery devices have been developed to support a patient in accurately and controllably delivering an amount of drug in a self-administration process.

Injection devices for self-administration usually comprise a dosing and dispensing mechanism allowing a user to dial a desired dose and subsequently to administer or dispense the dialed dose. The dispensing mechanism comprises a piston rod, which is movable relative to a housing of the injection device. The piston rod is adapted to move a plug inside the cartridge in a distal direction in order to dispense the drug through a distal opening of the cartridge.

The injection device further comprises the cartridge containing the liquid drug or medicament. The cartridge is usually hold by a cartridge holder and a housing of the injection device accommodating the dose and dispensing mechanism. To hold the cartridge a distal portion of the cartridge is accommodated in the sleeve-shaped cartridge holder and a proximal portion of the cartridge is accommodated in a recess of the housing. During assembly the cartridge is either first inserted with its proximal portion in the recess of the housing or it is first inserted with its distal portion in the cartridge holder. Subsequently, the cartridge holder is connected with its proximal end with a distal end of the housing. For dosing and dispensing accuracy it is important to rigidly and precisely hold the cartridge relative to the housing and the piston rod, in particular in an axial or dispensing direction.

Means for holding the cartridge within a housing are known in the art. For example, US 9,867,945 discloses a cartridge holder having inwardly and outwardly movable tongues on a proximal portion of the holder. The movable tongues comprise inwardly directed protrusion adapted to engage with a proximal rim portion of the cartridge. During insertion of the cartridge into the holder the tongues elastically bend outwards and flex back when the cartridge is inserted. Accordingly, in an assembled state the cartridge may be clamped between a distal end stop face of the holder and the protrusions such that the cartridge is secured against distal and proximal displacement with respect to the cartridge holder. EP 2311513 discloses arch-like deformation elements at a distal end of a cartridge housing.

US 9,149,580 discloses a cartridge holder comprising a tensioning member connected to a compression spring positioned at shoulder portion of a bottle-necked body of a cartridge. The cartridge holder is further equipped with at least one counter-bearing element adapted to provide a proximal end stop for the cartridge. Therefore, the cartridge can be effectively clamped between tensioning member and counter bearing element.

US 2007/0021718 discloses holding means for a cartridge comprising a spring inserted into a recess of a housing of a pen injector. A proximal end of the spring is held by a snap-fit connection in a bottom of the recess. When a cartridge is inserted with its proximal portion into the recess the spring is compressed. In the assembled state the compressed spring holds and biases the cartridge to a distal inner wall of the cartridge holder. The cartridge is thus axially hold relative to the housing and the cartridge holder.

US 9 132 237 B2 discloses a pen-type drug delivery device with cartridge. On a proximal side of the cartridge a spring washer is arranged within a housing which exerts a force on the cartridge in the distal direction and secures the cartridge against movement with respect to a cartridge retaining member and against movement with respect to a device housing. In order to ensure that the spring washer is kept in a range of elastic deformation, a further washer is arranged on the proximal side of the spring washer. The further washer limits the movement of the spring washer such that the spring washer is not plastically deformed.

US 2016/193414 A1 discloses a cassette unit for an injector for use with a syringe. The cassette unit comprises a housing having an end cap that is arranged for receipt of the syringe. The cassette unit further comprises an end cap spring defining a spring biasing relationship between the cassette unit end-cap and a flange of the syringe thereby urging the syringe forwards to hold the syringe within the cassette unit housing and to minimize any potential for the syringe to 'rattle about' within the cassette unit housing.

US 2017/0348489 A1 discloses an injections pen with a carpoule holder that can receive a carpoule. The carpoule holder is axially fixedly connected to a pen housing. In assembled state a holding element of the carpoule holder is elastically tensioned and pushed in the axial direction relative to a carpoule holder housing to fix and hold the carpoule relative to the housing.

These prior art approaches suffer from the fact that the holding means for holding the cartridge relative to the housing may exert a biasing force or holding force to the cartridge when inserting the cartridge into the housing which may unintentionally increase. Such a high holding force may occur if the cartridge is longer than expected (e. g. due to manufacturing tolerances) or if the cartridge is inserted during assembly to deep into the housing and thus biasing element is too much compressed. If the holding force is too high the cartridge may be damaged or even break.

On the other hand, if the holding force is too low the cartridge may move within the housing, in particular after a storage period when material relaxation has been occurred.

### DESCRIPTION OF THE INVENTION

It is thus an objective of the invention to reliably and precisely hold the cartridge relative to a housing of the drug delivery device or more general to provide an easy to manufacture alternative holding means with improved assembling capabilities.

This objective is achieved by a drug delivery device according to the independent claim Preferred embodiments are evident from the dependent patent claims.

According to the invention a housing for a drug delivery device for administrating a liquid drug from a cartridge comprises a recess for accommodating at least a proximal portion of the cartridge. The recess has an axis defining a longitudinal direction. The housing further comprises a deformable
member arranged inside the recess, wherein the deformable member comprises a proximal end attached to the housing, in particular to a side wall of the recess, and a distal end movable within the recess relative to the housing and relative to the side wall. The member is deformable from a non-deformed state or initial state to a deformed state by a displacement of the distal end. In the deformed state the member is adapted to limit a movement, in particular a proximal movement, of a cartridge present in the recess in the longitudinal direction relative to the housing. The member comprises a first arch between the proximal end and the distal end in the non-deformed state and in the deformed state. In the deformed state the first arch is deformed both elastically and plastically.

For an initial assembly the cartridge has to be inserted into the drug delivery device a proximal portion of the cartridge has to be inserted into the recess of the housing of the drug delivery device. During insertion of the cartridge into the recess the member is deformed from the non-deformed state to the deformed state. The member is deformable by a displacement of the distal end. The distal end is preferably displaced by a proximal end of the cartridge when the cartridge is inserted into the recess. When the cartridge has been inserted into a final axial position into the recess the arch is elastically deformed and at the same time plastically deformed. In this deformed state the housing with the member is adapted to limit a movement of the cartridge relative to the housing in the proximal longitudinal direction. Additionally, if the cartridge is inserted into its final axial position the member together with a cartridge holder or with any counter element connected to a distal end of the housing are adapted to hold the cartridge relative to housing such that the cartridge cannot move anymore relative to the housing.

During insertion of the cartridge a resisting force or reaction force exerted by the deformed member acts on the proximal end and in particular on a rim portion of the cartridge. There is no resisting force in the non-deformed state when the cartridge does not abut the member. The force appears and is continuously increasing at least in a first part during insertion of the cartridge into the recess because the arch of the member is deforming and some portions of the member are compressed. When the insertion of the cartridge is complete and the member is in its deformed state there is still a force exerted by the member acting on the cartridge because the member is in the deformed state not only plastically deformed but also elastically deformed.

The deformable member is adapted to limit a movement of the cartridge in the recess in the longitudinal direction relative to the housing. Therefore, the cartridge can be hold in a predefined final axial position or in a predefined final axial range in the recess relative to the housing. Due to the arch-shaped member the force progression in relation to the deflection or to the deforming path during deformation of the arch is optimal designable. For example, the arch can be designed such that the force progression is nearly linear or that it follows a suitable mathematic function. Thus, the force acting on the cartridge is very well predictable. Therefore, the member is adapted to limit the axial movement of an inserted cartridge reliably. In the assembled state, when a counter element, for example a cartridge holder, is mounted to the housing the cartridge can be reliably and precisely hold such that the cartridge is secured against distal and proximal displacement.

Since the force acting on the cartridge is very well adjustable by designing the member with an arch form the inserted cartridge can be hold with a predefined force within a predefined force range. That may be useful in order to be able to hold cartridges with different lengths, which may be caused due to manufacturing tolerances. Hence, the arch can be designed such that a short as well as a long cartridge can be optimally hold by the deformable member relative to the housing.

The elastic deformation of the arch causes a force acting on the proximal end of the inserted cartridge in a distal and longitudinal direction. The deformed arch biased the cartridge in a distal direction against a stop element in a cartridge holder or in any counter element in an assembled state.

During storage material relaxation may occur. Due to the elastic deformation of the arch there is still a remaining force acting on the cartridge and a movement of the cartridge can be reliably limited even if the initial force is reduced due to the material relaxation.

Besides the elastic deformation, the member is further plastically deformed in the deformed state such that a further movement of the cartridge is limited by the member. The effect of the plastic deformation is that depending on the design of the arch the exerted force on the cartridge by the member can be limited to a specific maximum value. Thus, the plastic deformation of the member can limit the exerted (reaction) force acting on the cartridge and can thus prevent a damage or even breakage of the cartridge.

In an assembled state when a holding means (e. g. a cartridge holder) are mounted the cartridge cannot move anymore or it can only move in a very restricted range (e. g. several micrometers) relative to the housing and to any dosing element such as a plunger rod accommodated in the housing. Thus, the accuracy of the dosing and dispensing is ensured.

In a preferred embodiment a sleeve shaped cartridge holder having a recess adapted to accommodate a distal portion of the cartridge is mounted to the housing in order to complete the mounting of the cartridge into the drug delivery device. Preferably, the opening of the recess of the cartridge holder is slide over a distal end of the cartridge. The cartridge is, as described above, with its proximal end inserted into the housing. Alternatively, the cartridge is first inserted with its distal portion in the cartridge holder and then the proximal portion of the cartridge is inserted into the housing as described above. In both variants, the proximal end of the cartridge holder is then fixed to the distal end of the housing, for example, by a snap fit connection. The cartridge is thus hold by the cartridge holder and the housing relative to the housing inside the drug delivery device.

Plastic deformation means that the deformation is irreversible and thus the deformation stays even after removal of the applied force. Elastic deformation means that the deformation is recoverable as it disappears after the removal of the applied force.

The term "arch" means that the member has a curved shape between the proximal end and the distal end. The arch or the curved shape may form an arc of, for example, in a range between 30° to 190°, preferable between 70° and 120°. The arch may be directed to any direction. The member may comprise one or more arches between the two ends.

In the deformed state the member is adapted to limit a movement of the cartridge in the longitudinal direction. That means movement of the cartridge is not stopped or blocked in any case but a movement of the cartridge is only possible in a restricted way. For example, the insertion force required for a movement of the cartridge in the proximal direction is higher than in the non-deformed state.

The term "non-deformed state" means the member is in its initial state after the manufacturing. The arch or any other portion of the member is not yet deformed by a user or by an assembly process, in particular by an insertion of the cartridge into the housing. The distal end of the member is not moved from its initial position.

In contrast, the term "deformed state" means at least a section of the member, in particular the shape of the arch has been altered from an initial shape to a new shape. In the deformed state the distal end of the member is moved away from its initial position, for example, from a distal position to a proximal position relative to the housing. The term "deformed state" is not limited to a specific deformation of the arch but the deformed state comprises all positions and orientations of the member and in particular of the arch different from their initial positions but in the deformed state the arch is deformed both elastically and plastically. In order to fulfill this limitation the deformation of the material of the member must be in the so-called strain hardening region of the stress-strain curve of the material. That means the member is in the deformed state as soon as the deformation of the material exceeds the pure (linear) elastic region and an irreversible plastic deformation occurs.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In a preferred embodiment the member is arm-shaped and the arch of the member is located between the proximal end and distal end of the arm-shaped member. The term "arm-shaped" means the member has preferably a longitudinal shape or form between the proximal end and the distal end of the member. Hence, the length is larger than the width of the member. However, the arm-shaped member must not be extending along the longitudinal direction of the housing but it may extend along any other direction or the whole member can have a curved form, which does not extend along a specific direction. The arm-shaped member enables to design an advantageous force procession of the force acting on the cartridge when the cartridge is inserted into the housing. Furthermore, the arm-shape allows an efficient production of the member. In case the member is made of plastic, the arm-shape is easy to demold from an injection molding tool.

Preferably, the member is deformable in the area of the arch. However, during deformation from the non-deformed state to the deformed state also other portion of the member may be deformed.

Preferably, the member is deformable from the non-deformed state to the deformed state by a displacement of the distal end of the member in the longitudinal direction relative to the housing, in particular by a proximal displacement. The displacement is preferably caused when the cartridge is inserted into the recess of the housing with its proximal portion and moved in the proximal direction parallel to the longitudinal axis. That means, during insertion of the cartridge along the longitudinal direction the distal end of the member is moved essentially along the longitudinal direction too. This allows an easy and quick deformation of the member and thus allows an easy and quick limitation of a movement of the cartridge relative to the housing.

Alternatively, the member may be deformable from the non-deformed state to the deformed state by moving the distal end in a direction perpendicular to the longitudinal direction or in any other direction.

In a preferred embodiment the distance between the proximal end and the distal end of the member defines a length of the member in the longitudinal direction, wherein in the deformed state the length is reduced compared to the non-deformed state. Preferably, the member and in particular the arch is bent or deformed such that the length of the member is reduced during deformation. Due to the deformation at least a portion of the arch is squeezed and/or stretched. The strain of the material due to the deformation causes a force acting at least partially in the longitudinal direction and is thus adapted to bias the inserted cartridge against a cartridge holder or any counter element at a distal end of the cartridge in an assembled state.

Alternatively, the length of the member is enlarged in the deformed state compared to the non-deformed state. This may occur, for example, if the arch of the member is stretched during deformation.

Preferably, in the deformed state a first portion of the arch is elongated and a second portion of the arch is compressed compared to the non-deformed state. Hence, the arch is deformed in such a way that an initial curve of the arch is further bent. For example, an inner radius defining an inner curvature of the arch may be decreased in the deformed state compared to the non-deformed shape. Such a deformation allows a space-saving design of the member. Furthermore, such a deformation allows a continuous and well predicable force progression in relation to the deforming path.

Alternatively, the arch may be deformed in any other way, such as a deformation only by elongation or only by compression.

In a preferred embodiment, the member comprises a second arch between the proximal end and the distal end and wherein the above already described first arch and the second arch constitute an s-shaped form in the non-deformed state and in the deformed state. The term "s-shaped" means the member has more or less an s-form. That means the first arch is preferably arranged adjacent to the second arch along a continuous line of the form of the member. The two arches of the member do not have to be exactly and completely form an s-curve. The s-shape form allows further a compact design. In the s-shaped member strain and stress within the body of the member are advantageously and widely dispersed within the body of the member and thus local strain peaks and local stress peaks can reliably avoided. Furthermore, during deformation the s-shape form of the member allows a continuous and optimal force progression in relation the deflection of the member.

The second arch allows a design of the deformable member such the resisting force or holding force of the deformable member during its deformation is smooth, continuous and can be held in an optimal range. Namely, the force in relation to the deflection (deforming path) of the s-shaped member is in a first section strong increasing and in a second section very low or almost constant. The almost constant section is desirable because a cartridge with a minimum length and a cartridge with a maximum length of a tolerance range can be hold with almost the same predetermined force.

Preferably, the member is deformable in the first and the section arch, in particularly exclusively deformable in the first and the second arch. The portions of the member not belonging to the first or second arch are thus preferably not deformed. Therefore, during deformation from the non-deformed state to the deformed state only the first arch and the second are deformed and the rest of the member is not deformed.

In an alternative embodiment the member can be designed such that only one of the two or more arches is deformed and the other arches are not deformed in the deformed state of the member.

Preferably, a plane area of the cross section of the member is varying between the proximal end and the distal end. The plane area of the cross section lies in a plane perpendicular to the center line of the member. In a prefered embodiment the member is tapered or the member comprises at least tapered portions. The varying plane area of cross section allows to define optimally the location of the deformable arch portion. Hence, the plane area in the deformable portion in the arch may be reduced compared with the non-deformable portion of the member such that the location of the deformation is precisely defined and predicable. Furthermore, the plane area in the deformable portion may be designed such that the portion is easily and optimally deformable.

In a preferred embodiment the member is unitary formed with the housing. Therefore, the member is formed in one piece and preferably with the same material as the housing. That allows an efficient production of the housing with the deformable member.

Alternatively, the deformable member may be a separate and single part, which is connected, for example, by adhesive bond or by a snap-fit connection.

Advantageously, the member is made of a polymer such as a polyoxymethylene, polypropylene, polyethylene, polycarbonate-acrylonitrile-butadiene-styrene, polybutylenterephthalat, a polyester or a polyamide. These polymers, in particular polyethylene, are easy deformable and have an advantageous value for the elongation at break. That means the deformable member can be deformed both plastically and elastically in a specific deformation range without risk of breakage. The material polypropylene is very ductile, which means the deformable member is easy to stretch or easy to deform.

In a preferred embodiment the housing comprises at least two deformable members as described above arranged inside the recess. The two or more members are preferably arranged circumferentially and preferably evenly and/or symmetrically spaced apart from each other. Thus a movement of a cartridge inserted into the recess of the housing can be limited by at least two and preferably by a plurality of members and the movement is thus limited in a safe and optimal manner.

Preferably, the proximal end of the member is attached to a connecting surface of the housing located inside the recess and essentially extending along the longitudinal direction and preferably in distal direction. That means, the member extends from the surface perpendicular to the insertion direction of the cartridge, which is along the longitudinal direction. The term "essentially" means that the connecting surface may not exactly be orientated in the longitudinal direction but may be orientated from about -10° to +10° different from the longitudinal direction.

The proximal end is preferably attached to the connecting surface on side wall and thus not in the space filled by the inserted cartridge. If the cartridge is inserted deeper into the recess than the final axial end position then the deformable member is deflected and the arch is further deformed but the movement of the cartridge is not blocked by the deformable member.

Preferably, the cartridge if further moved into the housing abuts an axial end stop element inside the housing, which is separated from the deformable member. Since the deformable member is attached to a surface extending along the longitudinal direction or to a sidewall of the recess the resisting force of the member can increase only to a specific maximum value. Hence, the cartridge cannot be damaged or broken by the member because the force acting on the cartridge cannot exceed the maximum value or a predefined threshold.

In contrast, a spring or any deformable element which is attached to a connecting surface perpendicular to the longitudinal direction (and thus perpendicular to the movement of an inserting cartridge) or a deformable element which is arranged on a proximal front end side of the inserted cartridge can only be deformed to a specific maximum and the resisting force caused by the member may abruptly increase to a breaking force when cartridge is inserted too deep into the recess.

In a preferred embodiment the connecting surface is an outer wall of an element inside the recess of the housing.

Such an outer wall can be, for example, an outer surface of an insert element arranged coaxially inside the recess, wherein the outer wall of the insert extends along the longitudinal direction. In this case, the member extends preferably radially outward from the outer wall in direction to the inner side wall of the recess.

In an alternative embodiment the connecting surface is an inner side wall of the recess. In this case, the members extends preferably radially inward from the inner side wall to a center of the recess.

The housing comprises preferably a stop element adapted to prevent a movement of the cartridge in the distal longitudinal direction beyond the stop element. The stop element is separate from the deformable member. That means the stop element is not a part or a portion of the deformable member. It can be unitary formed with the housing or it can be a separate element connectable to the housing.

The stop element is adapted to stop or block any movement of the cartridge in the proximal and longitudinal direction. In contrast, the deformable member is adapted to limit a movement of the cartridge in the proximal and longitudinal direction, which means a movement is not prevented per se, but it is restricted and the movement is dependent on forces acting on the cartridge in the longitudinal direction.

Since the stop element is separated from the deformable member the two function of limiting a movement (and/or biasing the cartridge) and providing an axial end stop are separated. This allows an optimal design of each function. That means the deformable member can be designed that the resisting force or holding force (caused by the deformed member) in relation of the deflection of the deformable member can be optimally adjusted to a specific cartridge length or range of lengths. Additionally, a stop end adapted to block a proximal movement of the cartridge inside the housing beyond a specific axial position can be provided independent of the deformable member in a space-saving design.

The invention relates further to a cartridge unit comprising a housing as described above, the cartridge containing the liquid drug, a cartridge holder for accommodating a distal portion of the cartridge and connectable to a distal end of the housing such that the cartridge is held relative to the housing by the cartridge holder and the housing. The member is preferably deformable from the non-deformed state to the deformed state by a displacement of the distal end of the member. The displacement is preferably caused by an insertion of a proximal portion of the cartridge into the housing wherein a proximal end of the cartridge abuts the distal end of the member. Therefore, in the deformed state the proximal end of the cartridge abuts the distal end of the member.

In a preferred embodiment and in an assembled state the distal portion of the cartridge is within the cartridge holder and the proximal portion of the cartridge is within the housing. Furthermore, a proximal end of the cartridge holder is connected to a distal end of the housing. Thus, the cartridge is hold within the cartridge holder and the housing because the member is in the deformed state and the deformed member limits a movement of the cartridge relative to the housing and the cartridge holder.

Furthermore, the invention relates to a drug delivery device for administrating a liquid drug from the cartridge comprising the cartridge unit as described above and a dosing and dispensing mechanism.

The disclosure relates further to a method for assembling a cartridge unit for a drug delivery device, the method comprising the steps of
a. Providing a housing comprising a recess for accommodating a proximal portion of the cartridge, the recess having an axis defining a longitudinal direction, a deformable member being arranged inside the recess, wherein the deformable member comprise a proximal end attached to the housing and a distal end movable within the recess relative to the housing, the member further comprising an arch;
b. Inserting a distal portion of the cartridge into an opening of a sleeve-shaped cartridge holder;
c. Inserting a proximal end portion of the cartridge holder with the cartridge into the recess of the housing in the longitudinal direction;
d. Deforming, by a proximal end of the cartridge, the deformable member of the housing from the non-deformed state to the deformed state by a displacement of a distal end of the member in the proximal and longitudinal direction and thereby deforming the arch both elastically and plastically;
e. Connecting the proximal end portion of the cartridge holder with a distal end portion of the housing, in particular by a snap lock.

The cartridge can be firstly inserted with its distal portion into the opening of the cartridge holder and secondly the cartridge holder with the inserted cartridge can be connected with the proximal end of the cartridge holder to the housing. Alternatively, the cartridge can be firstly inserted with its proximal portion into the opening of the housing and secondly the cartridge holder can be pulled over the distal portion of the cartridge and thirdly the proximal end of the cartridge holder can be connected to the housing.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an injection pen;
- Fig. 2a: depicts a sectional side view, wherein the cut runs along the longitudinal axis of the housing;
- Fig 2b: depicts a front view of the housing, without cartridge and cartridge holder;
- Fig. 3a-3d: depicts the deforming of the member from the non-deformed to the deformed state;
- Fig. 4: depicts a diagram showing the force progression in relation to the deflection of the member;
- Fig. 5: depicts a sectional side view of a housing for a smaller cartridge.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

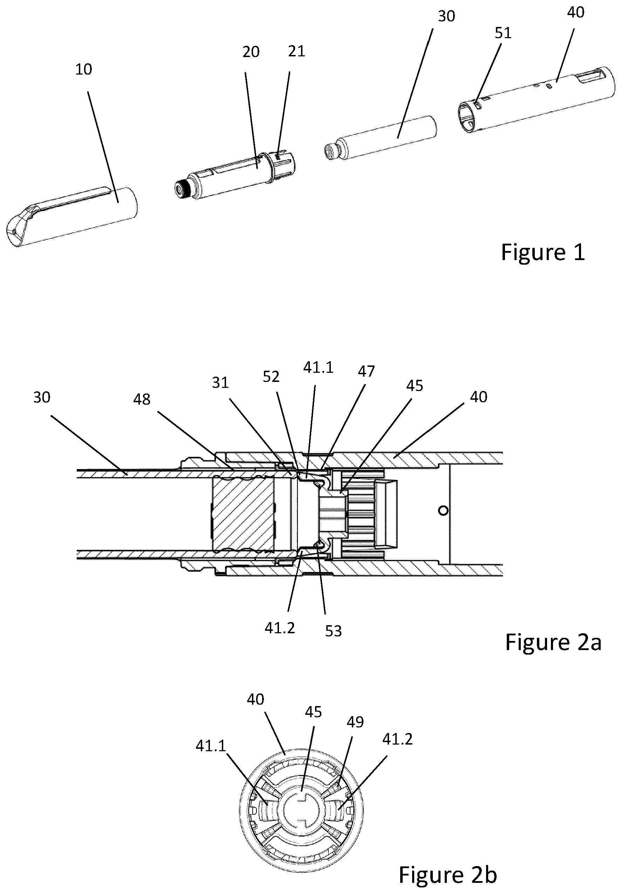

The following describes a drug delivery device with a cartridge unit and a housing according to the invention. The drug delivery device is in the described embodiment an injection pen. Figure 1 perspectively shows some parts of the injection pen, namely a housing 40, a cartridge 30, a cartridge holder 20 and a pen cap 10. The injection pen further comprises a dosing and dispensing mechanism which is not described further here. The housing 40, the cartridge 30 and the cartridge holder 20 form a cartridge unit. In the following the cartridge unit is described in detail. Firstly, the structural features are described, secondly, the functional features are depicted in detail.

Figure 2a depicts a sectional view of the cartridge unit, wherein the cut of the sectional view runs along the longitudinal axis of the housing 40. The housing 40 has a sleeve form and is made of polypropylene. In a proximal portion of the housing 40 the dosing and dispensing mechanism is accommodated. The housing 40 comprises a recess 48 at a distal end portion, which recess 48 is adapted to accommodate a proximal portion of the cartridge 30. The recess 48 has an essentially cylindrical shape. An opening forms a distal end of the recess 48 while a bottom made of four struts 49 circumferentially spaced apart from each other (see figure 2b) form the proximal end of the recess 48. The struts 49 support an insert 45 with a through hole in the center of the recess. The later serves as a guiding for an axially moveable piston rod (not shown) of the dosing and dispensing mechanism (not shown). An axis from the distal end to the proximal end of the recess 48 defines a longitudinal direction.

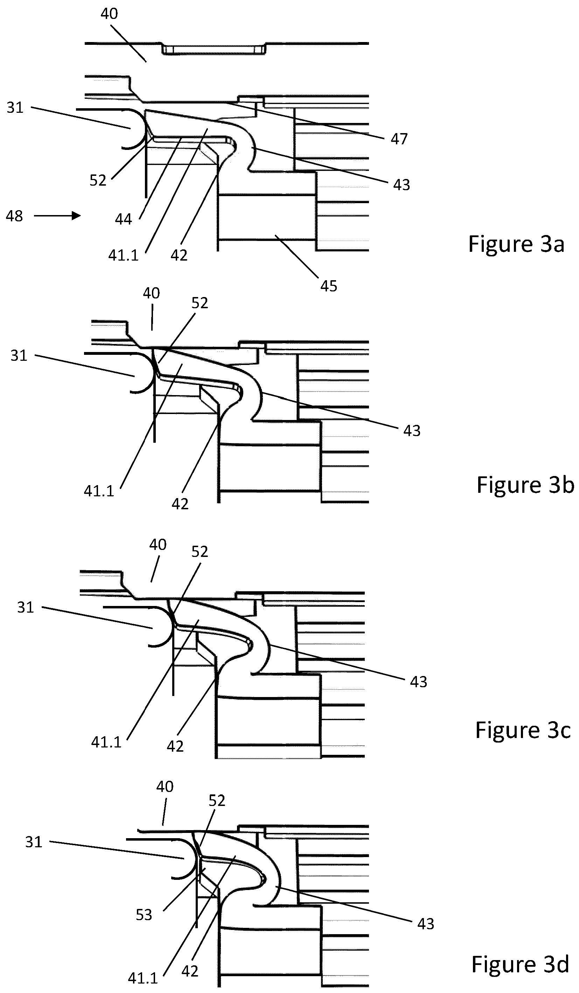

As it can be seen in figure 2a and 2b on an outer circumferential wall of the insert 45 two deformable members in the form of arms 41.1, 41.2 are attached. The two arms 41.1, 41.2 are circumferentially spaced apart from each other. A distal end 52 of each arm 41.1, 41.2 is moveable within the recess 48 relative to the housing 40 and a proximal end of each arm 41.1, 41.2 is inextricably attached to the outer wall of the insert 45. Each arm 41.1, 41.2 comprises a first arch 42 and a second arch 43 (see figure 3a-3d) which are arranged such that the arm has an s-shape. Starting from the attached proximal end of the arm the arm 41.1, 41.2 has a bend or curve by the first arch 42 from the wall of the insert 45 in proximal direction. The second arch 43 is adjacent the first arch and thus the arm 41.1, 41.2 has a bend or curve back in distal direction by the second arch 43. Each arm 41.1, 41.2 further comprises a straight section 44 at a distal portion of the arm 41.1, 41.2. The straight section 44 extends essentially along the longitudinal direction. The straight section 44 comprises a rib on its outer side (not shown) such that the cross section of the straight section is not rectangular. The distal end 52 of the arm is moveable relative to an inner side wall of the recess 48.

The plane area of the cross section of each arm 41.1, 41.2 varies between the distal end and the proximal end of the arm. Namely, the plane area of the cross section decreases from the distal end of the arm 41.1, 41.2 to the arches 42, 43 of the arm such that the plane area of the cross section in the s-shaped portion is reduced compared to the plane area of the cross section in a distal portion of the arm 41.1, 41.2.

On an inner side wall of the recess 48 there are two sliding surfaces 47 arranged along the longitudinal direction and circumferentially spaced apart from each other such that the sliding surfaces 47 are next to the distal end 52 of each arm 41.1, 41.2

The housing comprises further a end stop 53, which can stop or block a proximal moving of the cartridge beyond an end stop position. The end stop 53 comprises a stop surface that is arranged perpendicular to an insertion direction and which is arranged on each strut 49 at the bottom of the recess. When the cartridge is moved proximally beyond its final axial position it abuts the end stop 53 after a short movement in proximal direction. Hence, the end stop 53 prevents further insertion in proximal direction.

The cartridge holder 20 as shown in figure 1 is sleeve-shaped too and comprises a recess adapted to accommodate a distal portion of the cartridge 30. The cartridge holder 20 comprises latches or hooks 21 at its proximal end, which are adapted to engage with apertures 51 or counter elements arranged on a distal end of the housing 40. In the shown embodiment the cartridge holder 20 can be connected with its proximal end to the distal end of the housing by a snap lock connection. Alternatively, the cartridge holder 20 can be connected with the housing 40 by any other connecting means, for example, by adhesive bonding, by plastic welding, by a thread or by a bayonet fitting.

The function of the deformable arms 41.1, 41.2 and the interaction of the cartridge 30 with the housing and the cartridge holder 20 is described in the following with reference to figure 3a to 3d.

The figures 3a to 3d show a detailed and sectional view of the deformable arms 41.1, 41.2 of the housing 40. The cut of the sectional view runs along the longitudinal direction of the housing 40 and shows only the upper part of the section. In figure 3a an initial and non-deformed state of the deformable arms 41.1, 41.2 is depicted. When the cartridge 30 is inserted into the recess 48 with its proximal portion a proximal end or rim portion 31 of the cartridge 30 abuts a distal end 52 of the arms 41.1, 41.2. If the cartridge 30 is further inserted in proximal and in the longitudinal direction into the recess 48 of the housing 40 the rim portion 31 of the cartridge 30 moves the distal end 52 of the arm 41.1, 41.2 radially outward on a sliding surface 47 and slightly moves the distal end 52 in proximal direction, as shown in figure 3b. Consequently, an outer distal edge of the arm abuts the sliding surface 47 on the inner side wall of the recess 48 and the distal end 52 of each arm 41.1, 41.2 is further moved in proximal direction sliding along the surface 47. This movement deforms the two arches 42, 43 of each arm 41.1, 41.2. Due to the elastic deformation of the polypropylene material of the arms 41.1, 41.2 at this stage a reaction force or resisting force is caused by the deformation. The reaction force or resisting force acts on the rim portion 31 of the cartridge 30 and is essentially directed in distal direction.

Further insertion of the cartridge 30 in proximal direction moves the distal ends 52 of the arms 41.1, 41.2 further along the sliding surface 47 of the side wall of the recess 48 in the proximal direction and thereby further deforming the arches 42, 43 of each arm 41.1, 41.2. In particular, the first arch 42 is bent towards a center line or center of the recess 48. Due to that movement at least one of the two arches 42, 43 is deformed plastically. That means the deformation is irreversible and the deformation stays even after removal of the applied force by the cartridge insertion. From this instant, the arms 41.1, 41.2 are in the deformed state. That means in the deformed state the deformation of the material goes beyond a pure linear elastic deformation.

The position of the arms 41.1, 41.2 after the further insertion of the cartridge 30 in proximal direction is shown in figure 3c. At this stage, the first and the second arch 42, 43 are significantly deformed. The first arch 42 is bend or pressed towards a rigid outer wall of the insert 45 whereas the second arch 43 is rather widened or stretched.

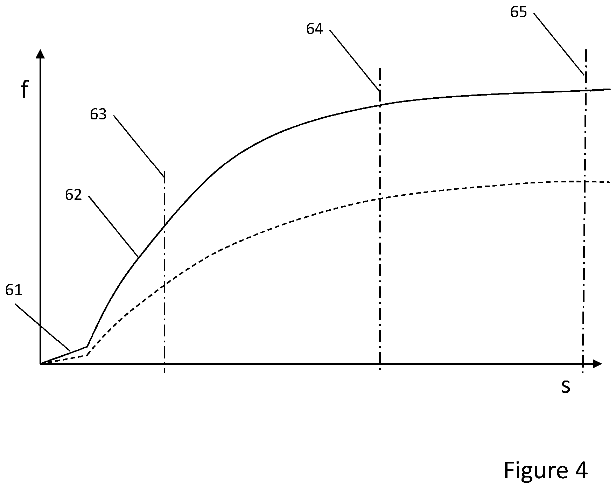

Figure 3d shows the final inserted position of the cartridge 30. The first and second arch 42, 43 of the arms 41.1, 41.2 are even more deformed than in the previous position shown in figure 3a to 3c. The deformation of the arms 41.1, 41.2 is still elastically and plastically and the arms 41.1, 41.2 are still in the deformed state. In case the cartridge 30 would be inserted even further into the recess in the longitudinal direction the reaction force would only slightly increase to a maximal value. However, the reaction force cannot exceed the maximal value due to the s-shape of the arms 41.1, 41.2. That effect will be described in detail with respect to the force-deflection diagram of figure 4. In this final position the rim portion 31 is near the end stop 53 but does not yet abut the end stop 53.

After the cartridge 30 is inserted into the recess 48 of the housing 40 up to a predefined axial position (inserted end position) the cartridge holder 20 is snapped with its latches or hooks 21 at the proximal end with the apertures 51 at the distal end of the housing 40. The cartridge 30 may be either first inserted with its distal portion into the recess of the cartridge holder and then the cartridge holder 20 with cartridge 30 may be connected to the housing 40 or the cartridge 30 may be first inserted with its proximal portion into the recess 48 of the housing 40 and then the cartridge holder 20 may be put over the distal end of the cartridge 30 and subsequently connected to the housing 40.

In figure 4 a force-deflection diagram is depicted, which shows the force progression of the reaction force (resistant force) of the arms 41.1, 41.2 in relation the deflection or deforming path of the arms (force vs deflection).

The horizontal axis (named as s-axis) shows the amount of deflection or the deformation path of the arms 41.1, 41.2 in the proximal and longitudinal direction when the distal end 52 of the arms 41.1, 41.2 is displaced from an initial distal position to a proximal position in the deformed state. The vertical axis (named as f-axis) shows the force progression of the reaction force or resisting force of the arms 41.1, 41.2.

A first section 61 of the curve in the diagram is nearly linear. This is at the very beginning of the deformation when the arm is bend radially outward (corresponding to the deformation shown in figure 3b) and the material of the arms 41.1, 41.2 is then in its linear elastic region. Further deflection of the arms - only axial movement of the distal end 52 of the arm 41.1, 41.2 in proximal direction is possible - abruptly increases the force. The curve in the diagram has a sharp bend and subsequently the curve rises strongly in second section 62 which is non-linear. From this point, the behavior is non-linear and not only an elastic but also a plastically deformation of the arms 41.1, 41.2 occurs at this stage. Thus, the arms 41.1, 41.2 have been transformed from the non-deformed state to the deformed state. That means at that stage the material is deformed not only elastically but also plastically.

Due to the arch-shaped arms the force-deflection curve has a predefined optimal procession. Namely, the maximal value of the force is chosen such that the cartridge is not damaged. Even if the cartridge is longer than expected or even if the cartridge is inserted too deep into the housing the resisting force or reaction force acting on the cartridge does not exceed a predefined maximum value. That means the force-deflection curve is flattened or nearly horizontal at a greater deflection of the arms (see left end section of the diagram).

The uninterrupted or solid line of the graph shows the behavior deflection vs force in with new molded polypropylene material. The dotted line of the graph shows the behavior if the polypropylene material is stored 10'0000 hours. Due to the material relaxation the resistant force is lower than with new material. However, with the s-shaped arms according to the invention the resisting force is still above a required minimum force to bias the cartridge against the cartridge holder in order to reliably hold the cartridge within the cartridge holder and the housing.

The vertical lines 63, 64 and 65 define the deflection range used for holding the cartridge. The range is broad in order to cover cartridges with different lengths. The left vertical line 63 defines the lower end of the range or a lower threshold with a minimum deflection if a short cartridge is held. The force generated by the deformed arms is still high enough to bias the short cartridge against the cartridge holder to reliable and safe hold the cartridge within the cartridge holder and the housing. The vertical line 64 in the middle of the deflection axis marks the nominal value 64 of deflection. The right vertical line 65 defines the upper end of the deflection for longer cartridges. The force generated by the deformed arms if inserted a long cartridge does not exceed a predefined value and thus even a long cartridge cannot be damaged.

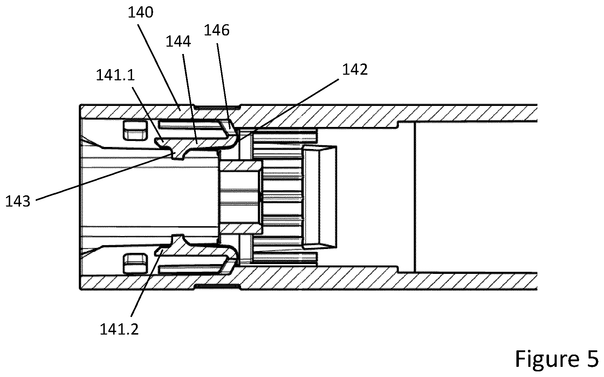

Figure 5 depicts a sectional view of a further embodiment of the invention. The housing 140 shown in figure 5 is adapted for cartridges with a smaller diameter, for example for a cartridge containing 1.5 ml of a drug, than the larger cartridges, for example for cartridges containing 3 ml of a drug, used for the housing 40 as described above with reference to figures 1 to 3.

In contrast to the above described embodiments of figure 1 to 3 the arms 141 are attached to an inner wall of the recess and not to the insert of the housing 140. Furthermore, the arms 141 comprise only one arch 142. The proximal end of the arm is attached to the inner wall of the recess and a first proximal section 146 of the arm 141 extents inclined in a proximal direction. The arch 142 follows the proximal section 146 such that a distal and straight section 144 of the arm 141 points in a distal direction. In the straight section 144 the arm 141 comprises a radially inwardly directed ledge 143 adapted to support the rim portion 31 of the inserted cartridge.

The insertion of a cartridge into the housing 140 and the deformation of the arms 144 corresponds to the embodiment described above with reference to figure 1 to 3. Namely, when inserting a cartridge the proximal end or rim portion of the cartridge abuts the ledge 143. Further insertion of the cartridge moves the distal end of the arms 144 in proximal direction. This leads to a deforming of the arches 142 and the arms 144 are deformed from the non-deformed state to the deformed state as described above with respect to the first embodiment.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 10: cap
- 20: cartridge holder
- 21: latch element
- 30: cartridge
- 31: rim portion
- 40: housing
- 41.1,41.2: arms
- 42: first arch
- 43: second arch
- 44: straight section
- 45: insert
- 46: outer wall of insert
- 47: sliding surface
- 48: recess
- 49: struts
- 51: apertures
- 52: distal end
- 53: end stop
- 61: linear section
- 62: non-linear section
- 63: left vertical line
- 64: nominal value line
- 65: right vertical line

- 140: housing
- 141.1, 141.2: arm
- 142: arch
- 143: ledge
- 144: straight section
- 146: proximal end section

## Claims

1. A drug delivery device for administrating a liquid drug from a cartridge (30), the delivery device comprising a housing, the cartridge and a cartridge holder adapted to hold the cartridge relative to the housing inside the drug delivery device, wherein the housing (40) comprising
a recess (48) for accommodating a proximal portion of the cartridge (30), the recess (48) having an axis defining a longitudinal direction;
a deformable member (41.1, 41.2) being arranged inside the recess (48),
the deformable member (41.1, 41.2) comprising a proximal end attached to the housing (40) and a distal end (52) movable within the recess (48) relative to the housing (40),
wherein the member (41.1, 41.2) is deformable from a non-deformed state to a deformed state by a displacement of the distal end (52), wherein in the deformed state the proximal end of the cartridge abuts the distal end of the deformable member (41.1.41.2) and wherein in the deformed state the member (41.1, 41.2) is further adapted to limit a movement of the cartridge (30) present in the recess (48) in the longitudinal direction relative to the housing (40),
**characterised in that**
the member (41.1, 41.2) comprises an arch (42, 43) and in the deformed state the arch (42, 43) is deformed both elastically and plastically.

2. The drug delivery device according to claim 1, **characterised in that** the member (41.1, 41.2) is arm shaped and wherein the arch (42, 43) is located between the proximal end and distal end (52) of the arm-shaped member (41.1, 41.2).

3. The drug delivery device according to claim 1 or 2, **characterised in that** the member (41.1, 41.2) is deformable from the non-deformed state to the deformed state by a displacement of the distal end (52) in the longitudinal direction relative to the housing (40).

4. The drug delivery device according to any of claims 1 to 3, **characterised in that** a distance between the proximal end and the distal end (52) defines a length of the member (41.1, 41.2) in the longitudinal direction, wherein in the deformed state the length is reduced compared to the non-deformed state.

5. The drug delivery device according to any of claims 1 to 4, **characterised in that** in the deformed state a first portion of the arch (42, 43) is elongated and a second portion of the arch is compressed compared to the non-deformed state.

6. The drug delivery device according to any of claims 1 to 5, **characterised in that** the member comprises a second arch (43), wherein the first arch (42) and the second arch (43) constitute a s-shaped form in the non-deformed state and in the deformed state.

7. The drug delivery device according to any of claims 1 to 6, **characterised in that** a plane area of the cross section of the member (41.1, 41.2) is varying between the proximal end and the distal end (52).

8. The drug delivery device according to any of claims 1 to 7, **characterised in that** the member (41.1, 41.2) is unitary formed with the housing (40).

9. The drug delivery device according to any of claims 1 to 8, **characterised in that** the member (41.1, 41.2) is made of a polymer selected from poly oxy methylene, polypropylene, polyethylene, polycarbonate-acrylonitrile-butadiene-styrene, polybutylenterephthalat, a polyester or a polyamide.

10. The drug delivery device according to any of claims 1 to 9, **characterised in** at least two deformable members (41.1, 41.2) arranged inside the recess (48).

11. The drug delivery device according to any of claims 1 to 10, **characterised in that** the proximal end is attached to a surface of the housing (40) located inside the recess (48) and essentially extending along the longitudinal direction.

12. The drug delivery device according to claim 11, **characterised in that** the surface is an outer wall of an element inside the recess (48) of the housing (40).

13. The drug delivery device according to claim 11 or 12, **characterised in that** the housing (40) comprises a stop element adapted to prevent a movement of the cartridge (30) in the proximal direction beyond the stop element, wherein the stop element is separate from the deformable member.

14. The drug delivery device according to any of claims 1 to 12; the cartridge (30) containing the liquid drug; a cartridge holder (20) for accommodating a distal portion of the cartridge (30) and connectable to a distal end of the housing (40) such that the cartridge (30) is held relative to the housing (40) by the cartridge holder (20) and the housing (40); wherein in the deformed state a proximal end of the cartridge (30) abuts the distal end (52) of the member (41.1, 41.2).

15. The drug delivery device according to claim 13 or 14 and a dosing and dispensing mechanism.

## Patentansprüche

1. Medikamentenabgabevorrichtung zum Verabreichen eines flüssigen Medikaments aus einer Patrone (30), die Abgabevorrichtung umfassend ein Gehäuse, die Patrone und einen Patronenhalter, der geeignet ist, um die Patrone relativ zu dem Gehäuse innerhalb der Medikamentenabgabevorrichtung zu halten, wobei das Gehäuse (40) umfasst
eine Aussparung (48) zum Aufnehmen eines proximalen Abschnitts der Patrone (30), wobei die Aussparung (48) eine Achse aufweist, die eine Längsrichtung definiert;
ein verformbares Element (41.1, 41.2), das innerhalb der Aussparung (48) angeordnet ist,
das verformbare Element (41.1, 41.2) umfassend ein proximales Ende, das an dem Gehäuse (40) befestigt ist, und ein distales Ende (52), das innerhalb der Aussparung (48) relativ zu dem Gehäuse (40) beweglich ist,
wobei das Element (41.1, 41.2) durch eine Verschiebung des distalen Endes (52) von einem nicht-verformten Zustand in einen verformten Zustand verformbar ist, wobei in dem verformten Zustand das proximale Ende der Patrone an dem distalen Ende des verformbaren Elements (41.1, 41.2) anliegt und wobei das Element (41.1, 41.2) in dem verformten Zustand ferner geeignet ist, um eine Bewegung der in der Aussparung (48) vorhandenen Patrone (30) in der Längsrichtung relativ zu dem Gehäuse (40) zu begrenzen,
**dadurch gekennzeichnet, dass**
das Element (41.1, 41.2) einen Bogen (42, 43) umfasst und in dem verformten Zustand der Bogen (42, 43) sowohl elastisch als auch plastisch verformt ist.

2. Medikamentenabgabevorrichtung nach Anspruch 1,
**dadurch gekennzeichnet, dass** das Element (41.1, 41.2) armförmig ist und wobei sich der Bogen (42, 43) zwischen dem proximalen Ende und dem distalen Ende (52) des armförmigen Elements (41.1, 41.2) befindet.

3. Medikamentenabgabevorrichtung nach Anspruch 1 oder 2,
**dadurch gekennzeichnet, dass** das Element (41.1, 41.2) durch eine Verschiebung des distalen Endes (52) in der Längsrichtung relativ zu dem Gehäuse (40) von dem nicht-verformten Zustand in den verformten Zustand verformbar ist.

4. Medikamentenabgabevorrichtung nach einem der Ansprüche 1 bis 3,
**dadurch gekennzeichnet, dass** ein Abstand zwischen dem proximalen Ende und dem distalen Ende (52) eine Länge des Elements (41.1, 41.2) in der Längsrichtung definiert, wobei in dem verformten Zustand die Länge im Vergleich zu dem nicht verformten Zustand reduziert ist.

5. Arzneimittelabgabevorrichtung nach einem der Ansprüche 1 bis 4,
**dadurch gekennzeichnet, dass** in dem verformten Zustand ein erster Abschnitt des Bogens (42, 43) gestreckt ist und ein zweiter Abschnitt des Bogens im Vergleich zu dem nicht verformten Zustand komprimiert ist.

6. Arzneimittelabgabevorrichtung nach einem der Ansprüche 1 bis 5,
**dadurch gekennzeichnet, dass** das Element einen zweiten Bogen (43) umfasst, wobei der erste Bogen (42) und der zweite Bogen (43) in dem nicht verformten und in dem verformten Zustand eine S-Form bilden.

7. Arzneimittelabgabevorrichtung nach einem der Ansprüche 1 bis 6,
**dadurch gekennzeichnet, dass** eine ebene Fläche des Querschnitts des Elements (41.1, 41.2) zwischen dem proximalen Ende und dem distalen Ende (52) variiert.

8. Arzneimittelabgabevorrichtung nach einem der Ansprüche 1 bis 7,
**dadurch gekennzeichnet, dass** das Element (41.1, 41.2) mit dem Gehäuse (40) einstückig ausgebildet ist.

9. Arzneimittelabgabevorrichtung nach einem der Ansprüche 1 bis 8,
**dadurch gekennzeichnet, dass** das Element (41.1, 41.2) aus einem Polymer hergestellt ist, ausgewählt aus Polyoxymethylen, Polypropylen, Polyethylen, Polycarbonat-Acrylnitril-Butadien-Styrol, Polybutylenterephthalat, einem Polyester oder einem Polyamid.

10. Arzneimittelabgabevorrichtung nach einem der Ansprüche 1 bis 9,
**gekennzeichnet durch** mindestens zwei verformbare Elemente (41.1, 41.2), die innerhalb der Aussparung (48) angeordnet sind.

11. Arzneimittelabgabevorrichtung nach einem der Ansprüche 1 bis 10,
**dadurch gekennzeichnet, dass** das proximale Ende an einer Oberfläche des Gehäuses (40) befestigt ist, die sich innerhalb der Aussparung (48) befindet und sich im Wesentlichen entlang der Längsrichtung erstreckt.

12. Arzneimittelabgabevorrichtung nach Anspruch 11,
**dadurch gekennzeichnet, dass** die Oberfläche eine Außenwand eines Bestandteils innerhalb der Aussparung (48) des Gehäuses (40) ist.

13. Arzneimittelabgabevorrichtung nach Anspruch 11 oder 12,
**dadurch gekennzeichnet, dass** das Gehäuse (40) einen Anschlagbestandteil umfasst, der geeignet ist, um eine Bewegung der Patrone (30) in der proximalen Richtung über den Anschlagbestandteil hinaus zu verhindern, wobei der Anschlagbestandteil von dem verformbaren Element getrennt ist.

14. Arzneimittelabgabevorrichtung nach einem der Ansprüche 1 bis 12; die Patrone (30), die das flüssige Medikament enthält; einen Patronenhalter (20) zum Aufnehmen eines distalen Abschnitts der Patrone (30), der mit einem distalen Ende des Gehäuses (40) derart verbindbar ist, dass die Patrone (30) durch den Patronenhalter (20) und das Gehäuse (40) relativ zu dem Gehäuse (40) gehalten wird; wobei in dem verformten Zustand ein proximales Ende der Patrone (30) an dem distalen Ende (52) des Elements (41.1, 41.2) anliegt.

15. Arzneimittelabgabevorrichtung nach Anspruch 13 oder 14 und ein Dosier- und Ausgabemechanismus.

## Revendications

1. Dispositif d'administration de médicament permettant d'administrer un médicament liquide à partir d'une cartouche (30), le dispositif d'administration comprenant un boîtier, la cartouche et un porte-cartouche adapté pour maintenir la cartouche par rapport au boîtier à l'intérieur du dispositif d'administration de médicament, dans lequel le boîtier (40) comprend
un évidement (48) permettant de loger une partie proximale de la cartouche (30), l'évidement (48) ayant un axe définissant une direction longitudinale ;
un élément déformable (41.1, 41.2) agencé à l'intérieur de l'évidement (48),
l'élément déformable (41.1, 41.2) comprenant une extrémité proximale fixée au boîtier (40) et une extrémité distale (52) mobile au sein de l'évidement (48) par rapport au boîtier (40),
dans lequel l'élément (41.1, 41.2) est déformable d'un état non déformé à un état déformé par un déplacement de l'extrémité distale (52), dans lequel, à l'état déformé, l'extrémité proximale de la cartouche vient en butée contre l'extrémité distale de l'élément déformable (41.1, 41.2) et dans lequel, à l'état déformé, l'élément (41.1, 41.2) est en outre adapté pour limiter un mouvement de la cartouche (30) présente dans l'évidement (48) dans la direction longitudinale par rapport au boîtier (40),
**caractérisé en ce que**
l'élément (41.1, 41.2) comprend un arc (42, 43) et, à l'état déformé, l'arc (42, 43) est déformé à la fois élastiquement et plastiquement.

2. Dispositif d'administration de médicament selon la revendication 1, **caractérisé en ce que** l'élément (41.1, 41.2) est en forme de bras et dans lequel l'arc (42, 43) est situé entre l'extrémité proximale et l'extrémité distale (52) de l'élément en forme de bras (41.1, 41.2).

3. Dispositif d'administration de médicament selon la revendication 1 ou 2, **caractérisé en ce que** l'élément (41.1, 41.2) est déformable de l'état non déformé à l'état déformé par un déplacement de l'extrémité distale (52) dans la direction longitudinale par rapport au boîtier (40).

4. Dispositif d'administration de médicament selon l'une quelconque des revendications 1 à 3, **caractérisé en ce qu'**une distance entre l'extrémité proximale et l'extrémité distale (52) définit une longueur de l'élément (41.1, 41.2) dans la direction longitudinale, dans lequel, à l'état déformé, la longueur est réduite par rapport à l'état non déformé.

5. Dispositif d'administration de médicament selon l'une quelconque des revendications 1 à 4, **caractérisé en ce qu'**à l'état déformé, une première partie de l'arc (42, 43) est allongée et une seconde partie de l'arc est comprimée par rapport à l'état non déformé.

6. Dispositif d'administration de médicament selon l'une quelconque des revendications 1 à 5, **caractérisé en ce que** l'élément comprend un second arc (43), dans lequel le premier arc (42) et le second arc (43) constituent une forme en S à l'état non déformé et à l'état déformé.

7. Dispositif d'administration de médicament selon l'une quelconque des revendications 1 à 6, **caractérisé en ce qu'**une surface plane de la section transversale de l'élément (41.1, 41.2) varie entre l'extrémité proximale et l'extrémité distale (52).

8. Dispositif d'administration de médicament selon l'une quelconque des revendications 1 à 7, **caractérisé en ce que** l'élément (41.1, 41.2) est formé d'un seul tenant avec le boîtier (40).

9. Dispositif d'administration de médicament selon l'une quelconque des revendications 1 à 8, **caractérisé en ce que** l'élément (41.1, 41.2) est constitué d'un polymère choisi parmi polyoxyméthylène, polypropylène, polyéthylène, polycarbonate-acrylonitrile-butadiène-styrène, polybutylène-téréphtalate, un polyester ou un polyamide.

10. Dispositif d'administration de médicament selon l'une quelconque des revendications 1 à 9, **caractérisé par** moins deux éléments
déformables (41.1, 41.2) agencés à l'intérieur de l'évidement (48).

11. Dispositif d'administration de médicament selon l'une quelconque des revendications 1 à 10, **caractérisé en ce que** l'extrémité proximale est fixée à une surface du boîtier (40) située à l'intérieur de l'évidement (48) et s'étendant essentiellement le long de la direction longitudinale.

12. Dispositif d'administration de médicament selon la revendication 11, **caractérisé en ce que** la surface est une paroi externe d'un élément à l'intérieur de l'évidement (48) du boîtier (40).

13. Dispositif d'administration de médicament selon la revendication 11 ou 12, **caractérisé en ce que** le boîtier (40) comprend un élément de butée adapté pour empêcher un mouvement de la cartouche (30) dans la direction proximale au-delà de l'élément de butée, dans lequel l'élément de butée est séparé de l'élément déformable.

14. Dispositif d'administration de médicament selon l'une quelconque des revendications 1 à 12 ; la cartouche (30) contenant le médicament liquide ; un porte-cartouche (20) permettant de recevoir une partie distale de la cartouche (30) et pouvant être relié à une extrémité distale du boîtier (40) de telle sorte que la cartouche (30) est maintenue par rapport au boîtier (40) par le porte-cartouche (20) et le boîtier (40) ; dans lequel, à l'état déformé, une extrémité proximale de la cartouche (30) vient en butée contre l'extrémité distale (52) de l'élément (41.1, 41.2).

15. Dispositif d'administration de médicament selon la revendication 13 ou 14 et un mécanisme de dosage et de distribution.
